# EUROPEAN PATENT SPECIFICATION

(11) **EP 1 324 977 B1**
(45) Date of publication and mention of the grant of the patent: **01.02.2006**
(21) Application number: 01972674.4
(22) Date of filing: 03.10.2001
(51) Int. Cl.: C07C 253/30, C07C 255/51

(54) **PROCESS FOR PRODUCING FLUORINATED DICYANOBENZENE**
VERFAHREN ZUR HERSTELLUNG VON FLUORIERTEM DICYANOBENZOL
PROCEDE RELATIF A L'ELABORATION DE DICYANOBENZENE FLUORE

(30) Priority: 04.10.2000 JP 2000305036; 21.12.2000 US 256915 P; 03.09.2001 JP 2001265573
(43) Date of publication of application: 09.07.2003
(73) Proprietor: Showa Denko K.K., Tokyo 105-8518 (JP)
(72) Inventor: SASAKI, Toru Higashinagahara Plant SHOWA DENKO K.K, Kawanuma-gun, Fukushima 969-3431 (JP); FURUKAWA, Tetsuhiro, c/o Higashinagahara Plant, Kawanuma-gun, Fukushima 969-3431 (JP); SATO, Yoshinori Higashinagahara Plant SHOWA D. KK, Kawanuma-gun, Fukushima 969-3431 (JP); YAMAMOTO, Takuji Higashinagahara Plant SHOWA D. KK, Kawanuma-gun, Fukushima 969-3431 (JP); YONEYAMA, Takashi Higashinagahara Plant SHOWA D.KK, Kawanuma-gun, Fukushima 969-3431 (JP)
(74) Representative: Strehl Schübel-Hopf & Partner
(86) International application number: PCT/JP2001/008718
(87) International publication number: WO 2002/028822

(56) References cited:
- EP-A- 0 120 575
- EP-A- 0 899 256
- WO-A-87/07267
- US-A- 3 290 353
- US-A- 3 975 424
- US-A- 5 153 350

## Description

### DETAILED DESCRIPTION OF THE INVENTION

### Technical Field

The present invention relates to a process for producing fluorinated dicyanobenzenes useful as an intermediate and raw material for preparation of medical and pharmaceutical products, agricultural chemicals and polymers. Particularly, tetrafluoroterephthalonitrile is important as an intermediate for agricultural chemicals.

### Background Art

As a process for producing fluorinated dicyanobenzenes represented by the formula (2): 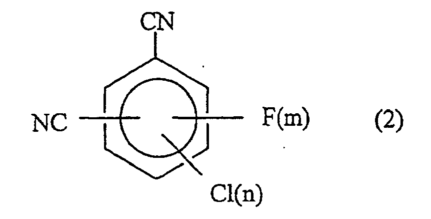
wherein m is an integer of 1 to 4, n is 0 or an integer of 1 to 3, and m + n = 4, a process for production of allowing a substituted dicyanobenzene to react with a fluorinating agent is known.

For example, JP-B-44-28493/1969 and Bull. Chem. Soc. Jpn, 40, 688(1971) disclose a process for producing tetrafluoro terephthalonitrile by allowing tetrachloroterephthalonitrile to react with potassium fluoride in the absence of a solvent. This process, however, has an extremely high reaction temperature of 300°C and a problem of corrosion of a reaction device. Further, it has a complicated process for isolating a product and a low yield of less than 80 %, so that it is difficult that this process is said to be an excellent process industrially.

JP-A-60-112751/1985 discloses a process for producing tetrafluorophthalonitrile by allowing tetrachloro phthalonitrile to react with a fluorinating agent in the presence of a benzonitrile solvent. The process has a high yield of from 90 to 92 %, but a high reaction temperature of about 300° C so that it has a problem of corrosion of a reaction device. Further, the process is required to complete the reaction for 10 hours or more and thereby this process is hardly said to be an advantageous process industrially.

In the meantime, JP-A-51-6940/1976 and USP3975424 disclose a process for preparing tetrafluoroterephthalonitrile by allowing tetrachloroterephthalonitrile to react with potassium fluoride in the presence of a polar solvent having a water content of not more than 0.2 %. This process has a low reaction temperature of 130°C and an excellent property such that the reaction is completed for a short time of 5 hours. This process, however, has a low yield of at most 81 %, and in the process, the solvent is used in an amount of 7.7 times by mass or more based on tetrachloroterephthalonitrile which is a raw material. Accordingly, in the case of carrying out the process industrially, it is expected that its productivity is low and large amounts of wastes would be discharged. The above prior arts, further, do not disclose a method of recovering the solvent used in the reaction nor a reaction device employed in carrying out the process industrially.

As described in the above, when carried out industrially, conventional processes for producing fluorinated dicyanobenzene have problems to be solved, such as its low yield and large amounts of industrial wastes.

### Object of the Invention

It is an object of the invention to provide a process for industrially producing a fluorinated dicyanobenzene represented by the formula (2): 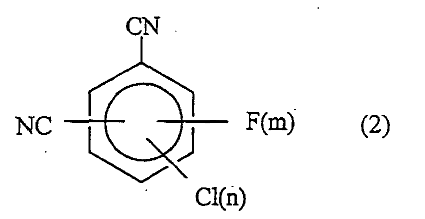 in the formula, m is an integer of 1 to 4, n is 0 or an integer of 1 to 3, and m + n = 4,
using, as a raw material, tetrachlorodicyanobenzene represented by the formula (1)

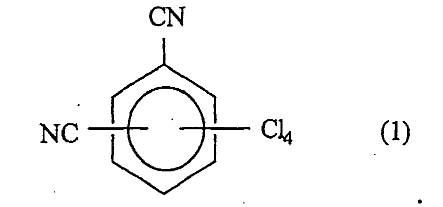

More specifically, in the processes for producing fluorinated dicyanobenzene by reacting tetrachlorodicyanobenzene with a fluorinating agent, it is an object of the invention to provide a process for producing a fluorinated dicyanobenzene in a high yield such that the reaction is carried out at a low temperature for a short time, which is unattained by conventional process.

### Means to Solve the Problems

The present inventors have reacted tetrachloro dicyanobenzene with a fluorinating agent using a non-protonic polar solvent and examined the effect of the amount of the non-protonic polar solvent on the reaction rate and the yield of fluorinated dicyanobenzene.

In result, the present inventors have been now found that decreasing the non-protonic polar solvent amount to less than 3 times by mass per tetrachlorodicyanobenzene, unexpectedly, fluorinated dicyanobenzenes can be produced not only at an improved reaction rate, but also at a higher yield at a higher purity, as compared with using the amount more than 3 times by mass.

Up to this time, decreasing the solvent amount based on the tetrachlorodicyanobenzene, the yield of fluorinated dicyanobenzene is occasionally lowered. The present inventors have found that decreasing the amount of the non-protonic polar solvent to less than 3 times by mass based on the tetrachlorodicyanobenzene, the reaction mixture is wet powdery or creamy, but not liquid, so that using conventional reaction vessels equipped with a stirrer, bulk solid matters are generated in the reaction mixture or adhered to the wall surface of the reaction vessel, and when the amounts thereof are increased, the yield of the aimed fluorinated dicyanobenzene is lowered. The inventors further have found that carrying out the reaction with disintegrating or removing the bulk solid matters, fluorinated dicyanobenzenes are preferably produced.

The present inventors have been accomplished the present invention on the basis of the above described investigation and findings.

Namely, the present invention comprises the following items.
1. A process for producing a fluorinated dicyanobenzene represented by the formula (2): 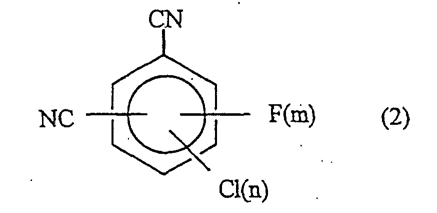
   wherein m is an integer of 1 to 4, n is 0 or an integer of 1 to 3, and m + n = 4,
   which process comprises allowing a tetrachlorodicyanobenzene represented by the formula (1) 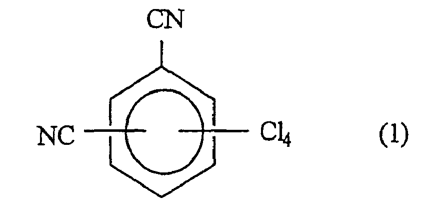
   to react with potassium fluoride in the presence of an organic solvent comprising at least one selected from the group consisting of N,N-dimethyl formamide, dimethyl sulfoxide and N-methyl-2-pyrrolidone in an amount of from 0.1 to 3 times by mass based on said tetrachlorodicyanobenzene, wherein said potassium fluoride is prepared by a spray drying method, and has an average bulk specific gravity of from 0.1 to 0.7 g/ml.
2. The process for producing a fluorinated dicyanobenzene according to item 1, wherein the solvent is N,N-dimethyl formamide.
3. The process for producing a fluorinated dicyanobenzene according to item 1 or 2, wherein the fluorinated dicyanobenzene represented by the formula (2) is tetrafluoro phthalonitrile, tetrafluoro isophthalonitrile or tetrafluoro terephthalonitrile.
4. The process for producing a fluorinated dicyanobenzene according to item 3, wherein the fluorinated dicyanobenzene represented by the formula (2) is tetrafluoroterephthalonitrile.
5. The process for producing a fluorinated dicyanobenzene according to any one of items 1 to 4, which process comprises carrying out the reaction while disintegrating bulk solid matters contained in a reaction mixture and/or while removing bulk solid matters adhered to a wall inside a reaction vessel.
6. The process for producing a fluorinated dicyanobenzene according to item 5, wherein, in carrying out the reaction while disintegrating bulk solid matters contained in the reaction mixture and/or while removing bulk solid matters adhered to the wall inside the reaction vessel, a mixing machine equipped with a ribbon-shaped and/or screw-shaped stirrer is used.
7. The process for producing a fluorinated dicyanobenzene according to item 5, wherein, in carrying out the reaction while disintegrating bulk solid matters contained in the reaction mixture and/or while removing the bulk solid matters adhered to the wall inside the reaction vessel, any one device of a kneader mixer, internal mixer, muller mixer, crusher, ribbon-shaped mixer, vertical screw-shaped (planetary-shaped) mixer and rotary mixer is used.
8. The process for producing a fluorinated dicyanobenzene according to any one of items 5 to 7, wherein the bulk solid matters are in an amount of not more than 10 % by mass based on the total amount of the reaction mixture in carrying out the reaction.
9. The process for producing a fluorinated dicyanobenzene according to any one of items 1 to 8, wherein the reaction temperature is between 80°C and 200°C.
10. A process for producing a fluorinated dicyanobenzene, which process comprises the steps of conducting a fluorinating reaction by the process as described in any one of items 1 to 9, thereafter cooling a reaction solution to lower than 60°C and adding water to crystallize and deposit a fluorinated dicyanobenzene represented by the formula (2).

### Mode of Carrying out the Invention

The present invention will be further described in detail hereinafter.

The tetrachlorodicyanobenzene represented by the formula (1) 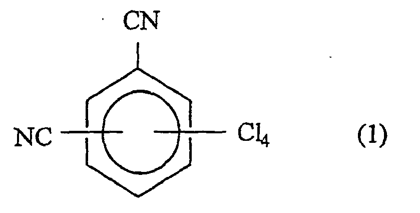
which is used as a raw material of the invention may include, for example, tetrachloroterephthalonitrile, tetrachloroisophthalonitrile and tetrachloroorthophthalonitrile.

The reaction process of the present invention is carried out by feeding tetrachlorodicyanobenzene, a non-protonic polar solvent as described in item 1. above in an amount of 0.1 to 3 times by mass based on the tetrachlorodicyanobenzene and a fluorinating agent as described in item 1. above to a reaction vessel and heating at a prescribed temperature with stirring. After the reaction, the mixture is crystallized and dried to prepare fluorinated dicyanobenzene of high purity in a high yield.

The fluorinated dicyanobenzene, which is a product of the present invention, is a compound of the formula (2) 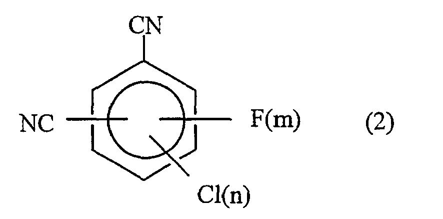
in which m is an integer of 1 to 4, n is 0 or an integer of 1 to 3, and m + n = 4, and may include, for example, trichlorofluorophthalonitrile, trichlorofluoroisophthalonitrile, trichlorofluoroterephthalonitrile, dichlorodifluorophthalonitrile, dichlorodifluoroisophthalonitrile, dichlorodifluoroterephthalonitrile, chlorotrifluorophthalonitrile, chlorotrifluoroisophthalonitrile, chlorotrifluoroterephtthalonitrile, tetrafluorophthalonitrile, tetrafluoroisophthalonitrile and tetrafluoroterephtalonitrile. Preferred examples may include tetrafluorophthalonitrile, tetrafluoroisophthalonitrile, chlorotrifluoroisophthalonitrile and tetrafluoroterephthalonitrile. Further preferred examples may include tetrafluoroterephthalonitrile.

The fluorinated dicyanobenzene, which is a product of the invention, can be mono-fluorine substituent, di-fluorine substituent, tri-fluorine substituent and tetra-fluorine substituent by regulating the amount of the fluorinating agent used in the reaction.

The fluorinating agent used in the present invention is potassium fluoride, preferably potassium fluorides prepared by the spray drying method (available from Morita Chemicals Inc.) having an average bulk specific gravity of from 0.1 to 0.7 g/ml because it has high reactivity.

The solvent used in the invention is N, N-dimethylformamide (DMF), dimethylsulfoxide (DMSO) or N-methyl-2-pyrrolidone or a mixture thereof.

In the reaction of the present invention, the non-protonic polar solvent is used in an amount of from 0.1 to 3 times by mass, preferably 0.1 to 2 times by mass based on tetrachlorodicyanobenzene which is a starting material. When the non-protonic polar solvent is less than 0.1 times by mass, the reaction rate is slow. When more than 3 times by mass, the yield of the resulting fluorinated dicyanobenzene is low.

The reaction of the present invention is carried out by thoroughly stirring the reaction mixture so that bulk solid matters are not produced in the creamy or wet powdery reaction mixture. During carrying out the reaction of the present invention, when the bulk solid matters are produced in the reaction mixture, or produced and adhered to the wall surface of the reaction vessel, it is preferred to carry out the reaction while disintegrating or removing these bulk solid matters. In the reaction of the present invention, it is preferred that the amount of bulk solid matters contained in the reaction mixture or adhered to the wall surface of reaction vessel be smaller.

The term "bulk solid matters" used in the specification means large solid matters, which are adhered to the inner wall of the reactor, or do not taken out and remained, after the liquid obtained by the reaction has been taken out from the reactor. The expression "removing" the bulk solid matters adhered to the wall surface of the reaction vessel means eliminating the bulk solid matters in the state fixed to the wall surface, for example, from the adhered surface, separating, peeling, scratching away or disintegrating. More specifically, examples of the procedure may include scratching away with a stirring blade and removing with application of force.

The mixer used as the reactor of the present invention is not particularly limited as long as it has a function of thoroughly stirring in such an extent that bulk solid matters are not produced in the creamy or wet powdery reaction mixture, or a function of disintegrating or removing the bulk solid matters produced or adhered to the wall surface of the reaction vessel and also has a heating mechanism.

As described in the above, the reactor used in the invention has no limitation as long as it has the function of disintegrating or removing the bulk solid matters, and further for attaining such a purpose it is preferably a device equipped with a stirrer having a ribbon-shaped or screw-shaped spiral blade.

Further, as the mixer having the heating mechanism suitable for the reaction process of the present invention, for example, a mixing device usable for viscosity materials and a device capable of mixing bulk solid materials are effective.

Examples thereof are a mixer designed in such a structure that the gap between the wall surface and the stirring blade is narrow, and a device of mixing while the screw revolves near the wall surface and also rotates. Specific examples thereof may include a kneader mixer, internal mixer, muller mixer, crusher, ribbon-shaped mixer, vertical screw-shaped mixer (planetary-shaped) [Nauta-mixer (Trade Mark Hosokawa Micron CO.) or the like] and rotary mixer.

The fact that these mixers are effective in the reaction of the present invention means that it is unnecessary in the reaction of the invention to enhance the stirring rate and make the whole reaction system uniform, as different from general reactions. This means that if only the mixing is conducted with disintegrating or removing bulk solid matters in order to not produce large amounts of bulk solid matters, the reaction proceeds. That is, it indicates that the reaction system has to only flow partly, but the whole does not need to be uniform. In usual reactions, even in solid-liquid reactions, it is a usual manner to conduct stirring so that the whole is uniform. Therefore, the reaction conditions of the present invention are those hardly expected from common knowledge.

The amount of the bulk solid matters produced in the reaction mixture or adhered to the wall surface of the reaction vessel according to the present invention is preferably not higher than 10 % by mass based on the whole reaction mixture. When the amount is over 10 % by mass, the reaction rate lowers, and further, the yield of fluorinated dicyanobenzene and the purity thereof lower.

In the case the reaction is continuously carried out with one reactor, if the amount of the bulk solid matters produced in the reaction mixture and adhered to the wall surface of the reaction vessel is not more than 10 % by mass based on the whole reaction mixture, the reaction may be carried out with the bulk solid matters remained inside the reactor and thereby particularly has no problems.

When the reaction mixture contains a large amount of water, there is such a problem that the reaction rate lowers and the yield lowers, so that the amount of the water is preferably smaller. Therefore, it is preferred that the water amount in the raw materials including the fluorinating agent, solvent and terachlorodicyanobenzene be smaller. Further, it is effective for the reaction to plan on the device to avoid moisture absorption or the like in feeding the raw materials, and to partly distill the solvent and then dehydrate after the feeding the raw materials. In some cases, it is also effective to distill and dehydrate by adding water-azeotropic other components.

In the present invention, the yield is increased by using the solvent in an amount of less than 3 times by mass based on the raw material tetrachlorodicyanobenzene. It is considered that one reason is based on that the moisture contaminated in the reaction system is easily depressed by decreasing the solvent amount used.

The reaction of the present invention is carried out at a reaction temperature of from room temperature to 200°C, preferably 80 to 200°C.

The reaction time varies depending to the reaction temperature or the kind of the objective fluorinated dicyanobenzene, and may be usually less than 10 hours.

In the present invention, the reaction may be carried out in the presence of a phase transfer catalyst. Examples of the phase transfer catalyst may include a quaternary phosphonium salt, quaternary ammonium salt, crown ether and polyalkylene glycol.

The fluorinated dicyanobenzene, which is the product of the present invention, can be isolated by, for example, crystallization, distillation, extraction, two-phase separation and sublimation. Among the methods, the crystallization of adding water into the reaction mixture after the reaction is effective. In the case of carrying out the crystallization with adding water, it is preferred that the reaction mixture be cooled to lower than 60°C before adding water because when the reaction solution temperature is high, the fluorinated dicyanobenzene product decomposes to decrease the yield and the purity of the resulting products. For example, in the preparation of tetrafluoroterephthalonitrile from tetrachloroterephthalonitrile and potassium fluoride in the presence of a N,N-dimethylformamide solvent, tetrafluoroterephthalonitrile having high purity of not less than 97 % can be easily isolated in such a way that the solution obtained after the reaction is cooled to lower than 60°C, then water is added in an amount of about 2.1 times by mass based on the used solvent to the solution, and the water solution is subjected to crystallization and filtration, and the resulting crystals are dried.

### Effect of the Invention

According to the present invention, fluorinated dicyanobenzenes having high purity useful as an intermediate and raw material for preparation of medical and pharmaceutical products, agricultural chemicals and polymers can be produced in a high yield by industrially profitable methods.

### Examples

The present invention will be described with reference to the examples hereinafter.

### <Analysis conditions>

The analysis conditions of liquid chromatography and gas chromatography employed in the examples are as follows.

### (Analysis conditions of liquid chromatography)

Device: Shimazu LC-10
Column: Shodex ODSpak F-511 (Particle diameter 5 µm, Size 4.6 mm (diameter) x 150 mm(length))
Eluting solution: Acetonitrile/water = 40/60
Flow rate: 1 ml/min
Detector: UV (242 nm)
Column temperature: 50°C
Injection: 5 µL

### (Analysis conditions of gas chromatography)

Device: Agilent 6890
Column: Agilent J&W DB-1 (Size 30 m (length) x 0.53 mm(diameter) x 1.5 µm (film thickness))
Carrier: Helium, 5.6 mL/min constant flow
Injection: Split (10:1), 1 µL, Inlet 300°C
Oven:
   (1) 80°C → 200°C (5°C/min)
   (2) 200°C→290°C (15°C/min)
   (3) 290°C (5 min)
Detector: FID 300°C
Internal standard substance: o-dichlorobenzene
Diluting solvent: Acetone

### Example 1

Into a 1 L cylindrical flask, 210 g of tetrachloroterephthalonitrile having a purity of 98.35 %, 205 g of potassium fluoride having an average bulk specific gravity of 0.5 g/ml prepared by the spray drying method and 475 g of N,N-dimethylformamide having a water content of 100 ppm as a solvent were charged, and while stirring them with a large-sized stirrer having ribbon blade in a nitrogen atmosphere, the temperature was elevated using an oil bath heated at 130°C. From the time at which the internal temperature reached to 115°C, the reaction was continued for 4.5 hr. Thereafter, the reaction solution was cooled to 60°C, and transferred into a 2 L round flask. The mass of bulk solid matters, which were adhered to the wall surface of the reactor during the reaction and remained after the transfer of the reaction solution, was 42.75 g. (this mass corresponds to 4.8 % of the all reaction solution mass). After the all amount of the reaction solution was transferred, 1018 g of water was introduced into the reaction solution with stirring over 40 min, and thereby the potassium chloride deposited were mostly dissolved and also crystals of tetrafluoroterephthalonitrile were deposited. The crystals were separated by a Nutsche funnel, and were washed with 400 g of water at 40°C. When analyzed by liquid chromatography and evaluated, the total amount of tetrafluorophthalonitrile present in the filtrate filtered and the washing water was 0.21 g. (this amount corresponds to 0.14 % of the yield based on tetrachloroterephthalonitrile). The resulting crystals were dried with a vacuum dryer at 60°C to obtain 146.2 g of dried crystals. When analyzed by gas chromatography, the crystals were found to be tetrafluoroterephthalonitrile having a purity of 99.1 %. The yield based on the tetrachloroterephthalonitrile was 93.2 %.

### Example 2

The procedure of Example 1 was repeated except the amount of N,N-dimethylformamide used was changed to 420 g. In result, 147.1 g of dried crystals of tetrafluoroterephthalonitrile having a purity of 99.0 % was prepared. The yield based on the tetrachloroterephthalonitrile was 93.7 %.

### Example 3

The procedure of Example 1 was repeated except the amount of N,N-dimethylformamide used was changed to 630 g. In result, 143.2 g of dried crystals of tetrafluoroterephthalonitrile having a purity of 99.0 % was prepared. The yield based on the tetrachloroterephthalonitrile was 91.2 %.

### Example 4

Into a 20 L glass reactor, 2.40 Kg of tetrachloroterephthalonitrile having a purity of 98.35 %, 2.34 Kg of potassium fluoride having an average bulk specific gravity of 0.5 g/ml prepared by the spray drying method and 5.43 Kg of N,N-dimethylformamide having a water content of 100 ppm as a solvent were charged, and while stirring them with a stirrer having turbine blade, the temperature was elevated using an oil bath. During the reaction, because bulk solid matters were adhered to the wall surface of the reactor, the reaction was carefully carried out with occasionally changing the velocity of the stirring so that the bulk solid matters were not adhered to the utmost. From the time at which the internal temperature reached to 115°C, the reaction was continued for 4.5 hr. Thereafter, the reaction solution was cooled to 60°C, and transferred into a 20 L vessel. When estimated from the mass of the reaction solution transferred, it was considered that the mass of bulk solid matters, which were adhered to the wall surface of the reactor during the reaction and remained after the transfer of the reaction solution, was about 1.01 Kg. (this mass corresponds to 9.9 % of the all reaction solution mass). After the all amount of the reaction solution was transferred, 11.63 Kg of water was introduced into the solution with stirring over 40 min, and thereby the potassium chloride deposited were mostly dissolved and also crystals of tetrafluoroterephthalonitrile were deposited. The deposited crystals were separated by a Nutsche funnel, and were washed with 2.28 Kg of water at 40°C. When analyzed by liquid chromatography and evaluated, the total amount of tetrafluoroterephthalonitrile present in the filtrate filtered and the washing water was 0.002 Kg. (the amount corresponds to 0.11% of the yield based on tetrachloroterephthalonitrile). The resulting crystals were dried with a vacuum dryer at 60°C to obtain 1.56 Kg of dried crystals. When analyzed by gas chromatography, the crystals were found to be tetrafluoroterephthalonitrile having a purity of 98.8 %. The yield based on the tetrachloroterephthalonitrile was 86.8 %.

### Example 5

Into a 2.5 m³ vertical screw-shaped (planetary-shaped) mixer, 420 Kg of tetrachloroterephthalonitrile having a purity of 98.35 %, 410 Kg of potassium fluoride having an average bulk specific gravity of 0.5 g/ml prepared by the spray drying method and 950 Kg of N,N-dimethylformamide having a water content of 100 ppm as a solvent were charged, and while stirring them with a stirrer at 60 rotations per minute and 0.8 revolution per minute in a nitrogen atmosphere, the temperature was elevated with passing steam at 3 Kg/cm² to a jacket. From the time at which the internal temperature reached to 115°C, the reaction was continued for 4.5 hr. Thereafter, the reaction solution was cooled to 60°C, and transferred into a 3.5 m³ crystallization bath using a slurry pump. Just after the reaction solution was transferred, 2036 Kg of water was introduced over 40 min, and thereby the potassium chloride deposited were mostly dissolved and also crystals of tetrafluoroterephthalonitrile were deposited. The deposited crystals were separated by a centrifugal filter, and were washed with 400 Kg of water at 40°C. Thereafter the crystals were dried with a conical dryer to obtain 292.3 Kg of dried crystals. When analyzed by gas chromatography, the crystals were found to be tetrafluoroterephthalonitrile having a purity of 99.0 %. The yield based on the tetrachloroterephthalonitrile was 93.1 %.

### Example 6

The procedure of Example 5 was repeated except that the amount of N,N-dimethylformamide used was changed to 760 Kg. In result, 296.1 Kg of dried crystals of tetrafluoroterephthalonitrile having a purity of 99.0 % was prepared. The yield based on the tetrachloroterephthalonitrile was 94.3 %.

### Comparative Example 1

Into a 20 L glass reactor, 2.03 Kg of tetrachloroterephthalonitrile having a purity of 98.35 %, 2.62 Kg of potassium fluoride having an average bulk specific gravity of 0.5 g/ml prepared by the spray drying method and 14.16 Kg of N,N-dimethylformamide having a water content of 100 ppm as a solvent were charged, and while stirring them with a stirrer having turbine blade, the temperature was elevated using an oil bath. From the time at which the internal temperature reached to 130°C, the reaction was continued for 5 hr. Thereafter, the reaction solution was introduced to a 50 L vessel containing 33.05 Kg of iced water. The potassium chloride deposited were mostly dissolved and also crystals of tetrafluoroterephthalonitrile were deposited. The deposited crystals were separated by a Nutsche funnel, and were washed with 1.73 Kg of water at 40°C. When analyzed by liquid chromatography and evaluated, the total amount of tetrafluorophthalonitrle present in the filtrate filtered and the washing water was 0.005 Kg. (the amount corresponds to 0.3 % of the yield based on tetrachloroterephthalonitrile). The resulting crystals were dried with a vacuum dryer at 60°C to obtain 1.22 Kg of dried crystals. When analyzed by gas chromatography, the crystals were found to be tetrafluoroterephthalonitrile having a purity of 99.0 %. The yield based on the tetrachloroterephthalonitrile was 80.4 %.

## Claims

1. A process for producing a fluorinated dicyanobenzene represented by the formula (2): 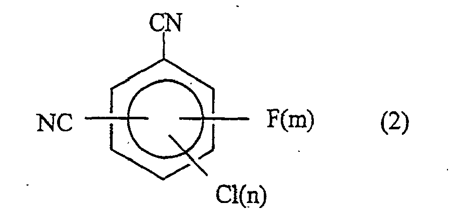
wherein m is an integer of 1 to 4, n is 0 or an integer of 1 to 3, and m + n = 4,
which process comprises allowing a tetrachlorodicyanobenzene represented by the formula (1) 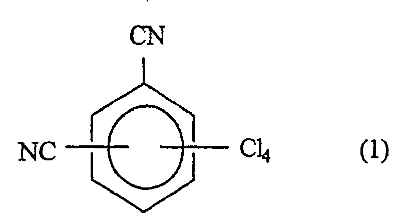
to react with potassium fluoride in the presence of an organic solvent comprising at least one selected from the group consisting of N,N-dimethyl formamide, dimethyl sulfoxide and N-methyl-2-pyrrolidone in an amount of from 0.1 to 3 times by mass based on said tetrachlorodicyanobenzene, wherein said potassium fluoride is prepared by a spray drying method, and has an average bulk specific gravity of from 0.1 to 0.7 g/ml.

2. The process for producing a fluorinated dicyanobenzene according to claim 1, wherein the solvent is N,N-dimethyl formamide.

3. The process for producing a fluorinated dicyanobenzene according to claim 1 or 2, wherein the fluorinated dicyanobenzene represented by the formula (2) is tetrafluoro phthalonitrile, tetrafluoro isophthalonitrile or tetrafluoro terephthalonitrile.

4. The process for producing a fluorinated dicyanobenzene according to claim 3, wherein the fluorinated dicyanobenzene represented by the formula (2) is tetrafluoroterephthalonitrile.

5. The process for producing a fluorinated dicyanobenzene according to any one of claims 1 to 4, which process comprises carrying out the reaction while disintegrating bulk solid matters contained in a reaction mixture and/or while removing bulk solid matters adhered to a wall inside a reaction vessel.

6. The process for producing a fluorinated dicyanobenzene according to claim 5, wherein, in carrying out the reaction while disintegrating bulk solid matters contained in the reaction mixture and/or while removing bulk solid matters adhered to the wall inside the reaction vessel, a mixing machine equipped with a ribbon-shaped and/or screw-shaped stirrer is used.

7. The process for producing a fluorinated dicyanobenzene according to claim 5, wherein, in carrying out the reaction while disintegrating bulk solid matters contained in the reaction mixture and/or while removing the bulk solid matters adhered to the wall inside the reaction vessel, any one device of a kneader mixer, internal mixer, muller mixer, crusher, ribbon-shaped mixer, vertical screw-shaped (planetary-shaped) mixer and rotary mixer is used.

8. The process for producing a fluorinated dicyanobenzene according to any one of claims 5 to 7, wherein the bulk solid matters are in an amount of not more than 10 % by mass based on the total amount of the reaction mixture in carrying out the reaction.

9. The process for producing a fluorinated dicyanobenzene according to any one of claims 1 to 8, wherein the reaction temperature is between 80°C and 200°C.

10. A process for producing a fluorinated dicyanobenzene, which process comprises the steps of conducting a fluorinating reaction by the process as claimed in any one of claims 1 to 9, thereafter cooling a reaction solution to lower than 60°C and adding water to crystallize and deposit a fluorinated dicyanobenzene represented by the formula (2).

## Patentansprüche

1. Verfahren zur Herstellung eines fluorierten Dicyanobenzols der Formel (2): 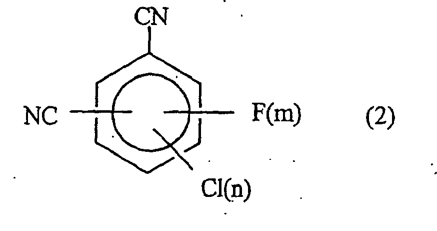
wobei m eine ganze Zahl von 1 bis 4 ist, n 0 oder eine ganze Zahl von 1 bis 3 ist, und m + n = 4,
wobei das Verfahren umfasst, ein Tetrachlorcyanobenzol der Formel (1) 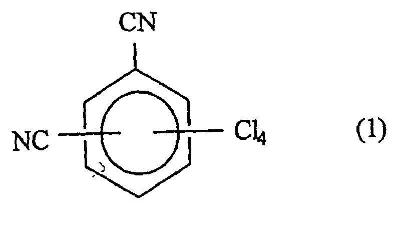
mit Kaliumfluorid in Gegenwart eines organischen Lösungsmittels, umfassend mindestens eines, das aus der Gruppe ausgewählt ist, bestehend aus N,N,-Dimethylformamid, Dimethylsulfoxid und N-Methyl-2-pyrrolidon, in einer Menge der 0,1- bis 3-fachen Masse, bezogen auf das Tetrachlordicyanobenzol, reagieren zu lassen, wobei das Kaliumfluorid durch ein Sprühtrocknungsverfahren hergestellt wird und eine durchschnittliche spezifische Schüttdichte von 0,1 bis 0,7 g/ml aufweist.

2. Verfahren zur Herstellung eines fluorierten Dicyanobenzols nach Anspruch 1, wobei das Lösungsmittel N,N-Dimethylformamid ist.

3. Verfahren zur Herstellung eines fluorierten Dicyanobenzols nach Anspruch 1 oder 2, wobei das fluorierte Dicyanobenzol der Formel (2) Tetrafluorphthalonitril, Tetrafluorisophthalonitril oder Tetrafluorterephthalonitril ist.

4. Verfahren zur Herstellung eines fluorierten Dicyanobenzols nach Anspruch 3, wobei das fluorierte Dicyanobenzol der Formel (2) Tetrafluorterephthalonitril ist.

5. Verfahren zur Herstellung eines fluorierten Dicyanobenzols nach einem der Ansprüche 1 bis 4, wobei das Verfahren das Durchführen der Reaktion umfasst, wobei feste, in dem Reaktionsgemisch enthaltene Bulkmaterialien abgebaut werden, und/oder feste Bulkmaterialien, die an einer innenseitigen Wand des Reaktionsgefäßes haften, entfernt werden.

6. Verfahren zur Herstellung eines fluorierten Dicyanobenzols nach Anspruch 5, wobei beim Durchführen der Reaktion unter Abbau von in dem Reaktionsgemisch enthaltenen, festen Bulkmaterialien und/oder unter Entfernung von festen Bulkmaterialien, die an einer innenseitigen Wand des Reaktionsgefäßes haften, eine Mischvorrichtung verwendet wird, die mit einem bandförmigen und/oder schrauben- bzw. schneckenförmigen Rührer ausgestattet ist.

7. Verfahren zur Herstellung eines fluorierten Dicyanobenzols nach Anspruch 5, wobei beim Durchführen der Reaktion unter Abbau von in dem Reaktionsgemisch enthaltenen, festen Bulkmaterialien und/oder unter Entfernung von festen Bulkmaterialien, die an einer innenseitigen Wand des Reaktionsgefäßes haften, irgendeine der folgenden Vorrichtungen eines Mischkneters, Innenmischers, Müller-Mischers bzw. Gegenstrom-Tellermischers mit Läufern und Schaufeln, Brechers, bandförmigen Mischers, vertikalschraubenförmigen (planetenförmigen) Mischers und einer Mischtrommel verwendet wird.

8. Verfahren zur Herstellung eines fluorierten Dicyanobenzols nach einem der Ansprüche 5 bis 7, wobei die festen Bulkmaterialien in einem Anteil von nicht mehr als 10 Massen-% vorliegen, bezogen auf die Gesamtmenge des Reaktionsgemisches, beim Durchführen der Reaktion.

9. Verfahren zur Herstellung eines fluorierten Dicyanobenzols nach einem der Ansprüche 1 bis 8, wobei die Reaktionstemperatur zwischen 80°C und 200°C beträgt.

10. Verfahren zur Herstellung eines fluorierten Dicyanobenzols, wobei das Verfahren die Schritte des Durchführens einer Fluorierungsreaktion durch das Verfahren nach einem der Ansprüche 1 bis 9 umfasst, anschließend Kühlen der Reaktionslösung auf niedriger als 60°C und Zugabe von Wasser, um ein fluoriertes Dicyanobenzol der Formel (2) zu kristallisieren und abzuscheiden.

## Revendications

1. Procédé de production d'un dicyanobenzène fluoré représenté par la formule (2) : 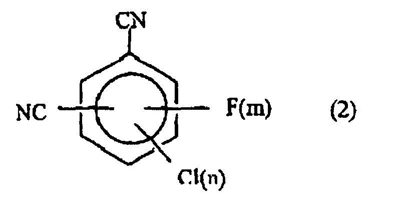
dans laquelle formule, m représente un nombre entier de 1 à 4, n vaut 0 ou représente un nombre entier de 1 à 3, et m + n = 4,
lequel procédé comprend le fait de laisser un tétrachlorodicyanobenzène représenté par la formule (1) 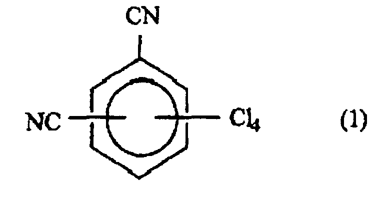
réagir avec du fluorure de potassium en présence d'un solvant organique comprenant au moins l'un choisi parmi du N,N-diméthylformamide, du diméthylsulfoxyde et de la N-méthyl-2-pyrrolidone, en une quantité valant de 0,1 à 3 fois la masse dudit tétrachlorodicyanobenzène, où ledit fluorure de potassium est préparé par un procédé de séchage par pulvérisation et possède une densité apparente moyenne valant de 0,1 à 0,7 g/ml.

2. Procédé de production d'un dicyanobenzène fluoré selon la revendication 1, dans lequel le solvant est du N,N-diméthylformamide.

3. Procédé de production d'un dicyanobenzène fluoré selon la revendication 1 ou la revendication 2, dans lequel le dicyanobenzène fluoré représenté par la formule (2) est du tétrafluorophtalonitrile, du tétrafluoroisophtalonitrile ou du tétrafluorotéréphtalonitrile.

4. Procédé de production d'un dicyanobenzène fluoré selon la revendication 3, dans lequel le dicyanobenzène fluoré représenté par la formule (2) est du tétrafluorotéréphtalonitrile.

5. Procédé de production d'un dicyanobenzène fluoré selon l'une quelconque des revendications 1 à 4, qui comprend la mise en oeuvre de la réaction tout en désintégrant des substances solides à l'état brut contenues dans le mélange réactionnel et/ou tout en éliminant des substances solides à l'état brut qui adhèrent à une paroi interne d'une cuve à réaction.

6. Procédé de production d'un dicyanobenzène fluoré selon la revendication 5, dans lequel, en effectuant la réaction tout en désintégrant des substances solides à l'état brut contenues dans le mélange réactionnel et/ou tout en éliminant des substances solides à l'état brut qui adhérent à la paroi interne de la cuve à réaction, on utilise un mélangeur équipé d'un agitateur en forme de ruban et/ou en forme de vis.

7. Procédé de production d'un dicyanobenzène fluoré selon la revendication 5, dans lequel, en effectuant la réaction tout en désintégrant des substances solides à l'état brut contenues dans le mélange réactionnel et/ou tout en éliminant des substances solides à l'état brut qui adhèrent à la paroi interne de la cuve à réaction, on utilise n'importe quel dispositif choisi parmi un mélangeur-malaxeur, un mélangeur interne, un mélangeur à meule, un broyeur, un mélangeur en forme de ruban, un mélangeur en forme de vis verticale (planétaire) et un malaxeur rotatif.

8. Procédé de production d'un dicyanobenzène fluoré selon l'une quelconque des revendications 5 à 7, dans lequel les substances solides à l'état brut sont présentes en une quantité d'au plus 10 % en masse par rapport à la quantité totale du mélange réactionnel lors de la mise en oeuvre de la réaction.

9. Procédé de production d'un dicyanobenzène fluoré selon l'une quelconque des revendications 1 à 8, dans lequel la température de réaction se situe entre 80°C et 200°C.

10. Procédé de production d'un dicyanobenzène fluoré, qui comprend les étapes consistant à effectuer une réaction de fluoration à l'aide du procédé selon l'une quelconque des revendications 1 à 9, refroidir ensuite la solution de la réaction à une température inférieure à 60°C et ajouter de l'eau afin permettre la cristallisation et le dépôt d'un dicyanobenzène fluoré représenté par la formule (2).
